# EUROPÄISCHE PATENTSCHRIFT

(11) **EP 1 239 910 B1**
(45) Veröffentlichungstag und Bekanntmachung des Hinweises auf die Patenterteilung: **01.03.2006**
(21) Anmeldenummer: 00991584.4
(22) Anmeldetag: 06.12.2000
(51) Int. Cl.: A61M 16/12

(54) **EXSPIRATIONSABHÄNGIGE GASDOSIERUNG**
EXPIRATION-DEPENDENT GAS DOSAGE
DOSAGE DE GAZ EN FONCTION DE L'EXPIRATION

(30) Priorität: 15.12.1999 DE 19960404
(43) Veröffentlichungstag der Anmeldung: 18.09.2002
(73) Patentinhaber: INO Therapeutics GmbH, 1111 Vienna (AT)
(72) Erfinder: MUELLNER, Rainer, A-2351 Wiener Neudorf (AT)
(74) Vertreter: Kasseckert, Rainer
(86) Internationale Anmeldenummer: PCT/EP2000/012244
(87) Internationale Veröffentlichungsnummer: WO 2001/043805

(56) Entgegenhaltungen:
- EP-A- 0 806 216
- WO-A-97/28838
- WO-A-98/31282
- US-A- 5 615 669

## Beschreibung

Die Erfindung betrifft ein Gasversorgungssystem mit einer gesteuerten Dosierung von mindestens einem Gas oder von mindestens einem Aerosol bei der Versorgung von Mensch oder Säugetier mit einem oder mehreren Gasen zur inhalativen Behandlung.

Geräte zur Beatmung werden eingesetzt für die maschinelle Beatmung, für die Anaesthesie und für die Atemtherapie durch Behandlung mit Gasen, z. B. Sauerstoffspende oder Behandlung mit Stickstoffmonoxid (NO).

Patienten mit chronischen Atembeschwerden (z. B. Asthma und COPD (chronisch obstruktive Atemwegserkrankung / Chronic Obstructive Pulmonary Disease)) werden durch einen in der Regel transportablen Sauerstoffspender in der Sauerstoffversorgung des Körpers unterstützt. Solche Patienten werden als spontanatmende Patienten bezeichnet, im Unterschied zu Patienten, die in der Klinik intubiert an ein Beatmungsgerät angeschlossen sind. Spontanatmende Patienten erhalten so zum Beispiel eine zusätzliche Sauerstoffspende (LOT = Langzeitsauerstofftherapie) oder eine Atmungsunterstützung (per CPAP = continuous posit. airways pressure). Verabreicht werden die Gase entweder über eine sogenannte Nasenbrille oder Nasensonde (Nasalapplikation; im einfachsten Fall ein Gasversorgungsschlauch, dessen Öffnung unterhalb der Nasenöffnungen des Patienten offen angeordnet ist) oder per Atemmaske (besonders bei CPAP).

In WO 98/31282 wird ein Gasversorgungssystem für beatmete oder spontanatmende Patienten beschrieben, bei dem ein oder mehrere Gase (z. B. NO, Sauerstoff) mittels einer Steuerung (Programmsteuerung, Sensorsteuerung oder kombinierte Programm/Sensorsteuerung) ungleichmäßig (kontinuierlich oder diskontinuierlich) in das Atemgas dosiert werden.

Die Gasdosierung bei den bekannten Gasversorgungssystemen erfolgt inspirationsgetriggert. Zwar ist aus der EP-A-0 806 216 eine Gase dosiervorrichtung bekannt, bei der während der Expiration ein Ventil getriggert wird, jedoch wird dabei das einem Atemgasgemisch zuzufuhrende Gas zunächst lediglich einer Dosierkammer und noch nicht dem Atemgasgemisch selbst zugeteilert. Wichtig ist bei der Triggerung der Gasdosierung das Maximum des inspiratorischen Flows, da meist zu diesem Zeitpunkt das dosierte Gas schon zur Verfügung stehen sollte. Dieses Maximum fällt im Regelfall mit dem Triggerstartpunkt zusammen. Durch mechanische, elektrische vor allem aber strömungstechnische Verzögerungen ist es nicht möglich den Beginn den tatsächlich in den Nasen-/Rachenraum dosierten Gasstrom mit dem maximalen inspiratorischen Flow gleichzusetzen.: Besonders das Totraumvolumen spielt eine große Rolle. Der anatomische Totraum umfaßt Nasen-Rachen-Raum, Trachea, Bronchien und Bronchiolen. Dieses Totraumvolumen beträgt beim Erwachsenen zwischen 150 und 200 ml. Außerdem wird ein Teil des in die Alveolen gelangenden A-temgases aufgrund einer Minderperfusion der betreffenden Alveolen nicht ausgenutzt. Dieser Totraum wird als alveolärer Totraum bezeichnet. Dieser Wert kann von Patient zu Patient stark schwanken. COPD-Patienten haben meist eine höhere Atemfrequenz dafür ein kleineres Atemzugsvolumen. Rechnet man mit einem Atemzugsvolumen von 400 ml und einem Totraumvolumen von 200 ml so macht das Totraumvolumen 50% des Atemzugsvolumens aus. Dadurch ist der therapeutische Effekt stark in Mitleidenschaft gezogen.

Bei der Inhalationstherapie sollte das Gas demnach so zugeführt werden, daß dieses möglichst in der gesamten Menge am Wirkort, dem Alveolärbereich, zur Verfügung steht.

Der Erfindung liegt die Aufgabe zugrunde, die Gasdosierung bei der Inhalationstherapie, insbesondere bei Spontanatmern, zu optimieren.

Gelöst wurde die Aufgabe durch ein Gasversorgungssystem mit den in Anspruch 1 beschriebenen Merkmalen.

Dem exspirationsgetriggerten Gasversorgungssystem gemäß der Erfindung liegt ein Gasversorgungssystem für beatmete oder spontanatmende Patienten zugrunde, wie es beispielsweise in WO 98/31282 beschrieben wird und worauf hiermit Bezug genommen wird. Das in WO 98/31282 beschriebene Gasversorgungssystem wird vorteilhaft modifiziert, wie im folgenden erläutert wird.

Das Gasversorgungssystem wird bei Mensch und Tier, insbesondere Säugetieren, eingesetzt.

Der Effekt des Bolus ("Gaspaket") wird ausgenutzt, so daß eine höhere Konzentration am Wirkort vorliegt (weit höher als die mittlere), da die Homogenisierung eine gewisse Zeit in Anspruch nimmt. Somit liegt dann auch ein höherer Partialdruckunterschied vor, was eine höhere Diffusion am Wirkort (z. B. Alveole) zur Folge hat. Hierzu ist es notwendig, den Beginn der inspiratorischen Phase möglichst genau zu kennen und auf oben erwähnte Effekte zu reagieren.

Das Therapiegas (z. B. O₂, NO) muß auf jeden Fall so zugeführt werden, daß es nicht im Totraum verbleibt, d.h. es muß auf jeden Fall am Gasaustausch teilnehmen bzw. diesen noch verbessern, in dem ein Bolus möglichst hochkonzentriert am Wirkort angelangt.

Das Therapiegas wird zu einem definierten Zeitpunkt vor dem Beginn der Inspiration dem Patienten zugeführt, um zu gewährleisten, daß das jeweilige Gas tatsächlich die Lungenareale erreicht, die es erreichen soll. Dazu ist es notwendig, den Verlauf der Exspiration zu kennen, um den Startpunkt der Dosierung genau zu definieren. Dies wird insbesondere durch eine Messung des Druckverlaufes während eines Atemzykluses (Exspiration und Inspiration), z. B. in der Nasenbrille, in der Regel mittels eines Drucksensors oder Flowsensors (oder darauf basierende Systeme) gewährleistet.

Der Druckverlauf ist für jeden Patienten charakteristisch. Da dieser Druckverlauf während jedes Atemzykluses relativ gleich ist, ist auf Grund eines augenblicklichen exspiratorischen Druckes zu ersehen, wann der Patient einatmen wird. D.h. es kann über einen Schwellenwert des entsprechenden Druckwertes der Zeitpunkt des Beginns der Inspiration vorhergesagt werden. Es wird also für jeden Patienten die Exspirationskurve aufgenommen, und mit Hilfe eines Algorithmus jedem Druckwert (abhängig davon ob die Kurve steigt oder fällt) ein bestimmter Zeitpunkt vor der Einatmung zugeordnet. Durch eine Aufnahme dieser Kurve patientenspezifisch durch den Arzt ist durch den Druckverlauf jeder Zeitpunkt der Exspiration genau definiert. Der Triggerimpuls wird also nicht durch den beim Einatmen entstandenen Unterdruck ausgelöst, sondern durch einen einstellbaren Überdruckschwellenwert resultierend aus dem Exspirationsverlauf. Bei der Triggerung während der Exspiration paßt sich die Triggerung den Patientenbedürfnissen durch den eventuell schwankenden Exspirationsverlauf an, da nicht mit einer Zeitkonstanten getriggert wird, sondern mit dem patientenabhängigen Überdruck in der Exspirationsphase. Dadurch kann eine automatische Anpassung der Triggerung abhängig von der Atemkurve des Patienten gewährleistet werden. Die Triggerung paßt sich also bei einer Mehrbelastung des Patienten, die auch zu einer Veränderung der Atemkurve führt, selbständig den veränderten Verhältnissen an. Dadurch ist es möglich die Dosierung von einem oder mehreren Therapiegasen so zu steuern, daß aufgrund der gegebenen, individuellen Physiologie des Patienten diverse Lungenareale gezielt vom Therapiegas angeströmt werden.

Weiters besteht die Möglichkeit verschiedenartige Gase zu verschiedenen Zeitpunkten während der Exspiration zu dosieren. Diese Zeitpunkte werden durch die Druckkurve genau definiert. Dadurch ist es möglich pro Atemzug verschiedene Lungenareale mit verschiedenen Gasen bzw. Gaskonzentrationen zu versorgen.

Dieses Verfahren kann für alle Gase die zur Therapie von Lungenkrankheiten vorteilhaft herangezogen werden.

Vor allem Patienten, die auf Grund Ihres Krankheitsbildes (z. B. Lungenfibrose) bisher auf eine kontinuierliche O₂-Zufuhr angewiesen waren, können mit diesem System auf eine gepulste Dosierung, und damit verbunden mit einem geringerem O₂-Verbrauch, umgestellt werden, wobei die Blutgaswerte sich im gleichen Rahmen wie bei der kontinuierlichen Gasversorgung bewegen.

Ein weiteres Anwendungsgebiet des Verfahrens ist z. B. eine Gas- oder Aerosoltherapie im Nasen Rachen-Raum oder in der Luftröhre. Das bedeutet hier, daß der Wirkbereich nicht direkt in der Lunge liegt sondern im anatomischen Totraum.
Dies wird vorteilhaft ebenfalls durch eine in die Exspiration gelegte Dosierung erreicht und kann genau gesteuert werden.

Die Erfindung wird anhand der Zeichnung erläutert.

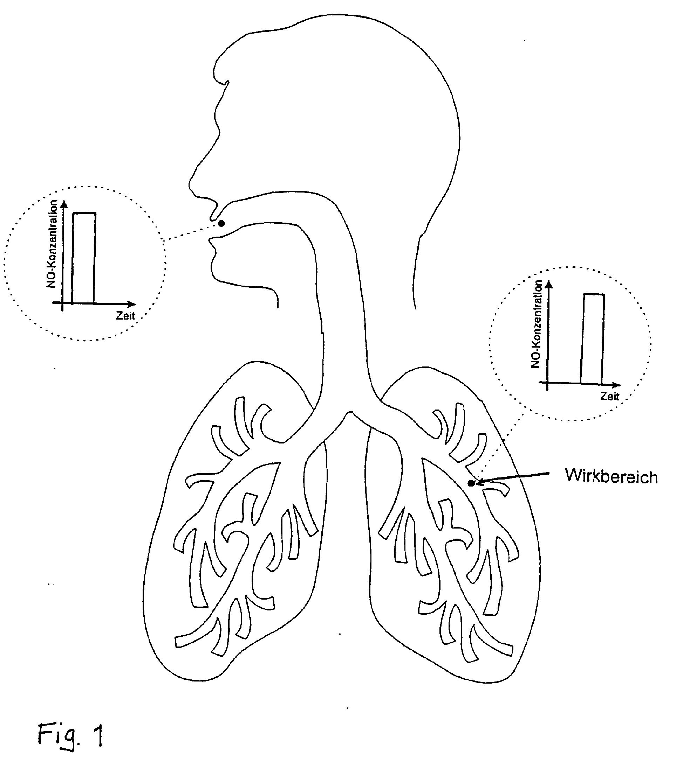

Fig. 1 veranschaulicht die Wirkung der exspirationsgetriggerten Gasdosierung, wobei ein Gasstoß (Bolus) des dosierten Gases an den Wirkort, z. B. in der Lunge des Patienten, gelangt.

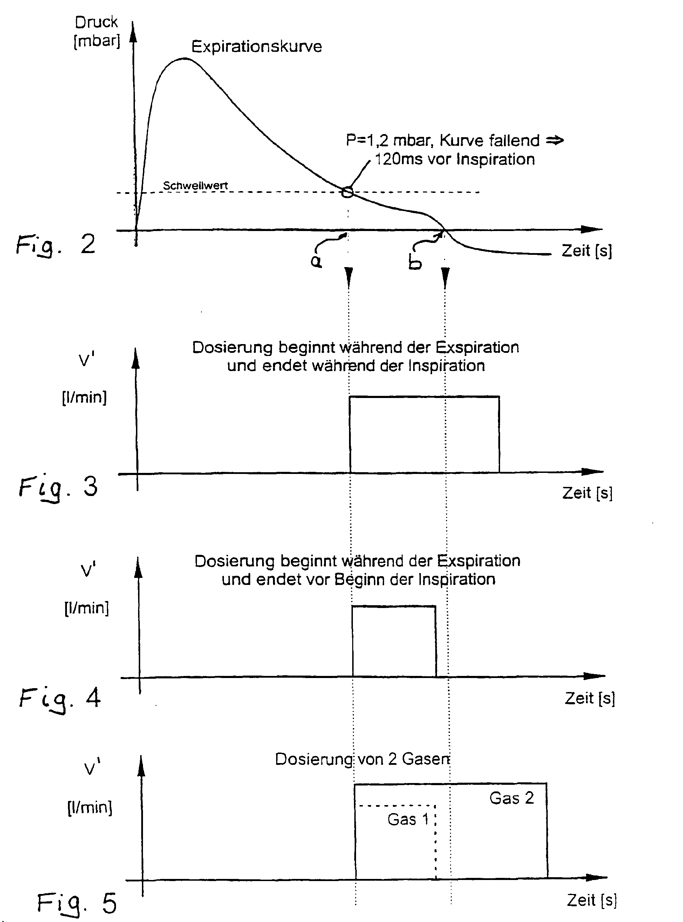

Fig. 2 zeigt schematisch eine am Patienten vor oder während der Gasbehandlung aufgenommene Exspirationskurve, bei der der mittels eines Sensors (z. B. vor der Nase oder in einer Atemmaske) aufgenommene Druck p (in mbar) in Abhängigkeit von der Zeit t (in Sekunden, s) dargestellt. Die Marke a stellt den Zeitpunkt des Erreichens eines festgesetzten Schwellenwertes des Druckes p und die Marke b den Zeitpunkt des Beginns der Inspiration dar.
Fig. 3 bis 5 zeigen schematisch den Volumenstrom V (in I/min.) von dosiertem Gas (z. B. Sauerstoff) in Abhängigkeit von der Zeit t (in Sekunden, s) für unterschiedliche Dosierintervalle. Die in Fig. 3 gezeigte Gasdosierung beginnt zum Zeitpunkt a während der Exspiration und endet nach dem Beginn der Inspiration, dem Zeitpunkt b, während der Inspiration. Die in Fig. 4 gezeigte Gasdosierung beginnt zum Zeitpunkt a während der Exspiration und endet vor dem Beginn der Inspiration, vor dem Zeitpunkt b. Fig. 5 zeigt die Dosierung von zwei Gasen, wobei die in Fig. 4 und Fig. 3 gezeigten Arten der Gasdosierung kombiniert sind.

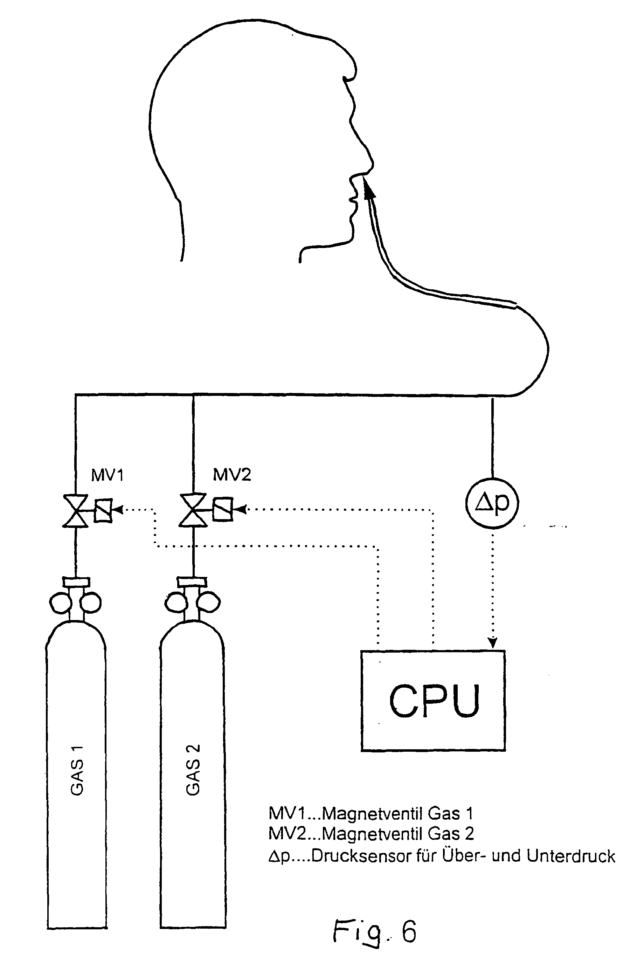

Fig. 6 zeigt ein Schema eines Gasversorgungssystems. Das Gasversorgungssystem ist für die Dosierung zweier Gase (Gas 1 und Gas 2) ausgelegt, die beispielsweise in Druckgasbehältern bereitgestellt werden. Die Gasdosierung erfolgt über die mit einer Steuereinheit (CPU) verbundenen steuerbaren Magnetventile (MV1 und MV2) in eine Gasleitung, die zum Patienten führt. Ein Drucksensor (bezeichnet mit Δp) für Über- und Unterdruck ist in der Gasleitung oder z. B. am Ausgang der Gasleitung (z. B. vor der Nase des Patienten) angeordnet.

Fig. 1 zeigt, wie durch ein definiertes Verhältnis zwischen Gasflow, Dosierzeit und dem entsprechenden Startpunkt der Dosierung während der Expiration jeder beliebige Ort in den Atemorganen gezielt therapiert werden kann. Vor allem durch kurze Gasdosierstöße (Bolus) können höhere Konzentrationen am Wirkort erreicht werden, ohne daß andere Bereiche davon in Mitleidenschaft gezogen werden. Dadurch kann sowohl der Gasverbrauch reduziert werden, was wiederum zu kleineren und damit leichteren Vorratsbehältern führt, als auch eventuelle Therapienebeneffekte minimiert werden. Durch die kurze Zeit der Dosierung kommt es zu keiner Homogenisierung des Gasgemisches und der Dosierstoß pflanzt sich bis zum gewünschten Wirkbereich fort (Fig. 1).

Ein Beispiel für eine Exspirationskurve als Grundlage für die Triggerung und Steuerung eines Dosierablaufes zeigt Fig. 2. Erreicht der Exspirationsdruck P bei fallenden Druckwerten den vorgegebenen oder während der Beatmung ermittelten Schwellenwert von 1,2 mbar, so wird die Dosierung (im Beispiel entspricht dies einer Zeit von 120ms vor dem Beginn der Inspiration) ausgelöst, wobei alle möglichen Formen der Dosierung (siehe Fig. 3, 4, 5) erfolgen können.

Durch eine Dosierung von Gasen bzw. Aerosolen nur während der Exspiration kann der anatomische Totraum gezielt angeströmt werden. Es kann gezielt z. B. der Nasen Rachen-Raum oder die Luftröhre behandelt werden (Fig. 3).

Die Dosierung kann sowohl über eine Nasenbrille oder über eine Atemmaske erfolgen.

Der Druckverlauf wird vorteilhaft durch den selben Drucksensor aufgenommen, der auch für das Auslösen des Triggersignals verantwortlich ist (Fig. 6).

Der Dosierablauf wird im folgenden erläutert.
Zu einem bestimmten Zeitpunkt der Exspiration (Marke a) wird eine gewisse Gas- oder Aerosolmenge dosiert. Die Dosierung kann entweder nur während der Exspiration weiter erfolgen (Therapie im anatomischen Totraum) oder auch während der Inspiration weiter erfolgen (Fig. 3, 4). Weiters können auch mehrere Gase dosiert werden (Fig. 5), wobei der Startpunkt der Dosierung (in Fig. 3 bis 5 die Marke a) nicht zwingend gleich sein muß. Die Dosiermengen und Dosierzeiten hängen dabei stark von der jeweiligen Therapie ab, und können beliebig variiert werden.

Je nach den Lungenarealen, die angetrömt werden sollen, variiert der Startpunkt der Dosierung, die Dauer der Dosierung, als auch die Dosiermenge.

## Patentansprüche

1. Gasversorgungssystem zur Gasbehandlung von Mensch und Tier mit einer gesteuerten Dosierung von mindestens einem Gas oder von mindestens einem Aerosol, **gekennzeichnet durch** eine Steuerung für eine Dosierung, bei der die Zuführung des Gases oder Aerosols zu einem Atemgas zu einem definierten Zeitpunkt während der Exspiration eines Patienten beginnt.

2. Gasversorgungssystem nach Anspruch 1, **dadurch gekennzeichnet, dass** der Bestimmung der Zeit des Beginns der Zuführung des Gases oder Aerosols eine aufgenommene Atemkurve oder Exspirationskurve zugrunde liegt.

3. Gasversorgungssystem nach Anspruch 1 oder 2, **dadurch gekennzeichnet, dass** das Gasversorgungssystem mindestens einen Drucksensor zur Steuerung und/oder Triggerung der Gasdosierung enthält.

4. Gasversorgungssystem nach einem der vorhergehenden Ansprüche 1 bis 3, **dadurch gekennzeichnet, dass** das Gasversorgungssystem programm- oder programm- und sensorgesteuert ist und eine Steuereinheit enthält.

5. Gasversorgungssystem nach einem der vorhergehenden Ansprüche 1 bis 4, **dadurch gekennzeichnet, dass** das Gasversorgungssystem eine Steuerung für eine automatische Anpassung der Triggerung der Gasdosierung, abhängig von der Atemkurve des Patienten, enthält.

## Claims

1. Gas supply system for gas treatment of humans and animals, with controlled dosing of at least one gas or of at least one aerosol, **characterized by** a dosage control in which the delivery of the gas or aerosol to a respiratory gas starts at a defined time during the expiration of a patient.

2. Gas supply system according to Claim 1, **characterized in that** the determination of the time for starting the delivery of the gas or aerosol is based on a recorded breathing curve or expiration curve.

3. Gas supply system according to Claim 1 or 2, **characterized in that** the gas supply system includes at least one pressure sensor which controls and/or triggers the dosing of the gas.

4. Gas supply system according to one of the preceding Claims 1 to 3, **characterized in that** the gas supply system is program-controlled, or is program- and sensor- controlled, and includes a control unit.

5. Gas supply system according to one of the preceding Claims 1 to 4, **characterized in that** the gas supply system includes a control means which automatically adapts the triggering of the gas dosing as a function of the breathing curve of the patient.

## Revendications

1. Système d'alimentation en gaz pour le traitement à l'aide de gaz de personnes et d'animaux avec un dosage commandé d'au moins un gaz ou d'au moins un aérosol, **caractérisé par** une commande pour un dosage dans laquelle l'apport du gaz ou de l'aérosol à un gaz respiratoire commence à un instant défini pendant l'expiration d'un patient.

2. Système d'alimentation en gaz selon la revendication 1, **caractérisé en ce que** la détermination de l'instant du début de l'apport du gaz ou de l'aérosol dépend d'une courbe respiratoire ou d'une courbe expiratoire enregistrée.

3. Système d'alimentation en gaz selon la revendication 1 ou 2, **caractérisé en ce que** le système d'alimentation en gaz contient au moins un capteur de pression pour la commande l'oxyde d'éthylène le déclenchement du dosage de gaz.

4. Système d'alimentation en gaz selon l'une quelconque des revendications précédentes 1 à 3, **caractérisé en ce que** le système d'alimentation en gaz est commandé par programme ou par programme et capteur et contient une unité de commande.

5. Système d'alimentation en gaz selon l'une quelconque des revendications 1 à 4, **caractérisé en ce que** le système d'alimentation en gaz contient une commande pour une adaptation automatique du déclenchement du dosage de gaz, en fonction de la courbe respiratoire du patient.
